# EUROPEAN PATENT APPLICATION

(11) **EP 3 643 578 A1**
(43) Date of publication of application: **29.04.2020**
(21) Application number: 18820795.5
(22) Date of filing: 24.05.2018
(51) Int. Cl.: B61L 3/02, A61B 17/43, C12M 3/00

(54) **PIPETTE FOR RETAINING OVOCYTES IN INTRACYTOPLASMIC SPERM INJECTION METHODS**

(30) Priority: 21.06.2017 ES 201730743 U
(71) Applicant: Vergara Alcaide, Francisco, 18150 Gójar (ES)
(72) Inventor: Vergara Alcaide, Francisco, 18150 Gójar (ES)
(74) Representative: Munoz Garcia, Antonio
(86) International application number: PCT/ES2018/070375
(87) International publication number: WO 2018/234600

(57) **Abstract**

The present invention relates to a pipette for holding oocytes in intracytoplasmic sperm injection procedures without prior cytoplasm aspiration, configured from a long and narrow cylindrical body (10) provided with a working end (1a) for holding the oocyte (2) by means of pressure, having a decreasing frustoconical portion (11) where the diameter thereof is significantly reduced until defining a small support tip (12) having a planar, straight, and angled configuration with respect to the cylindrical body (10). The support tip (12) is prolonged into a short segment forming an angle (a) between 20 and 30° with the axial axis of the cylindrical body (10).

## Description

### OBJECT OF THE INVENTION

As expressed in the title of the present specification, the invention relates to a pipette for holding oocytes in intracytoplasmic sperm injection procedures providing the intended function thereof with advantages and novelty features that are described in detail below and entail a significant improvement of the current state of the art.

More specifically, the object of the invention lies in a micropipette the purpose of which is to serve as means for holding the oocyte in intracytoplasmic sperm injection or ICSI procedures, the novel structural configuration of which, unlike conventional pipettes being used up until now to that end and acting by means of suction, prevents the step of cytoplasm aspiration with the injection needle that is required with said conventional pipettes to assure the breaking of the cytoplasmic membrane, given that the new pipette, provided with a planar and angled end, holds the oocyte by means of a slight pressure which allows breaking the cytoplasmic membrane without performing said suction.

### FIELD OF APPLICATION OF THE INVENTION

The field of application of the present invention is comprised in the sector of the industry dedicated to the manufacturing of medical instruments, focusing particularly on the field of microdevices applicable for processes and interventions on the cellular level, and more specifically to those intended for use in assisted reproduction processes.

### BACKGROUND OF THE INVENTION

As is known, intracytoplasmic sperm injection or ICSI is an assisted reproduction technique consisting of the fertilization of oocytes by injecting a spermatozoid into their cytoplasm by means of a micropipette, after obtaining and preparing the gametes for the purpose of obtaining embryos that can be transferred to the mother's womb.

To carry out said technique, the oocyte previously removed from the cumulus must be held with a holding pipette, whereas the spermatozoid is injected with another injection pipette.

The problem is that the membrane surrounding the oocyte is a membrane that is hard to break due to its high elasticity, in fact, the injection needle can be introduced into the cytoplasm without actually breaking the membrane (microscopically, invagination of the membrane will occur with respect to the injection pipette but the membrane does not break). The conventional way of breaking the membrane is to perform aspiration with the injection pipette until breaking the membrane. However, this leads to a disorganized cytoplasm that may critically affect the mitotic spindle (a situation that the present inventors considered as only probable, given that it is not observed with the routine ICSI technique) if the arrangement thereof is altered during cytoplasm aspiration.

The breaking of the membrane is required for the proper release of the spermatozoid into the cytoplasm, as well as for activating the Ca++ or calcium ion channel mechanisms that in turn intervene in the activation of cell division mechanisms.

The objective of the present invention is therefore to develop a new type of holding pipette which fulfills said function of holding the oocyte to enable breaking its membrane without aspirating the cytoplasm.

Moreover and as a reference to the current state of the art, it must be indicated that at least the applicant itself is unaware of the existence of any other similar pipette with the same or similar application for holding oocytes in intracytoplasmic sperm injection procedures having technical, structural, and constitutive features that are equal or similar to those of the pipette herein claimed.

### SUMMARY OF THE INVENTION

Therefore, the pipette proposed by the invention is configured as a remarkable novelty in its field of application, since the aforementioned objectives are successfully and unquestionably attained as a result of its implementation, with the characterizing details making this possible and suitably distinguishing it being suitably included in the final claims attached to the present description.

Specifically, as described above, the invention proposes a pipette for holding oocytes in intracytoplasmic sperm injection procedures which fulfills said holding function without cytoplasm aspiration, as it prevents such aspiration step with the injection needle which is required with conventional holding pipettes to assure the breaking of the cytoplasmic membrane and cause the activation of the oocyte and more readily deposit the spermatozoid in the cytoplasm, and which, however, as indicated in the preceding section, may cause damage in the original structure of the cytoplasm, and in some cases may alter the structure of the mitotic spindle.

To that end, the holding pipette of the present invention is configured from a long and narrow cylindrical body, the working end of which, i.e., the end applied for holding the oocyte, instead of being rounded like the end of the conventional holding pipettes, has a decreasing frustoconical portion and a planar tip which is prolonged after the decreasing portion, forming an angle between 20 and 30° with the axial axis of the cylindrical body, said planar tip being configured in said manner to support and hold the oocyte cell by means of applying a slight pressure.

The holding pipette of the invention is made of glass tested safe for embryos, similarly to the conventional holding pipettes, and its manufacturing method is also similar to that of the conventional holding pipettes.

In turn, the dimensions of the pipette and of its parts are designed so that they can be used in the same microinjection equipment in which the conventional holding pipettes are used, varying the protocol of the ICSI technique only slightly.

The pipette for holding oocytes of the invention therefore holds the oocyte by means of a slight pressure, not by means of suction like conventional pipettes. This slight pressure in turn allows breaking the cytoplasmic membrane without having to perform cytoplasm aspiration, increasing the turgor of the oocyte, thereby further facilitating the breaking of the membrane.

The described pipette therefore represents an innovation in structural and constitutive features unknown up until now, and these reasons combined with its practical usefulness provide it with sufficient grounds to be granted the exclusive privilege that is sought.

### DESCRIPTION OF THE DRAWINGS

To complement the description that is being made and for the purpose of helping to better understand the features of the invention, a set of drawings is attached to the present specification as an integral part thereof in which the following has been depicted in an illustrative and non-limiting character:
Figure 1 shows a top plan view of the working end of a holding pipette according to the prior art, depicted along with the L and the injection pipette that are used for ICSI processes herein described, where the rounded configuration of said conventional pipette can be seen.
Figure 2 shows a side elevational view of the holding pipette object of the invention, where its general configuration and the main parts it comprises can be seen.
Figures 3 and 4 show respective enlarged side elevational and plan views, respectively, of the working end of the pipette of the invention shown in Figure 2, where the configuration of its decreasing area and its planar and angled tip can be seen in greater detail.
Figures 5 and 6 shows respective schematic views of the way of operation of the working end of the pipette of the invention for holding the oocyte without cytoplasm aspiration.

### PREFERRED EMBODIMENT OF THE INVENTION

In view of the mentioned drawings and according to the numbering used, there can be observed therein an example of a holding pipette according to the prior art and an non-limiting example of the pipette of the invention, which comprises the parts and elements indicated and described in detail below.

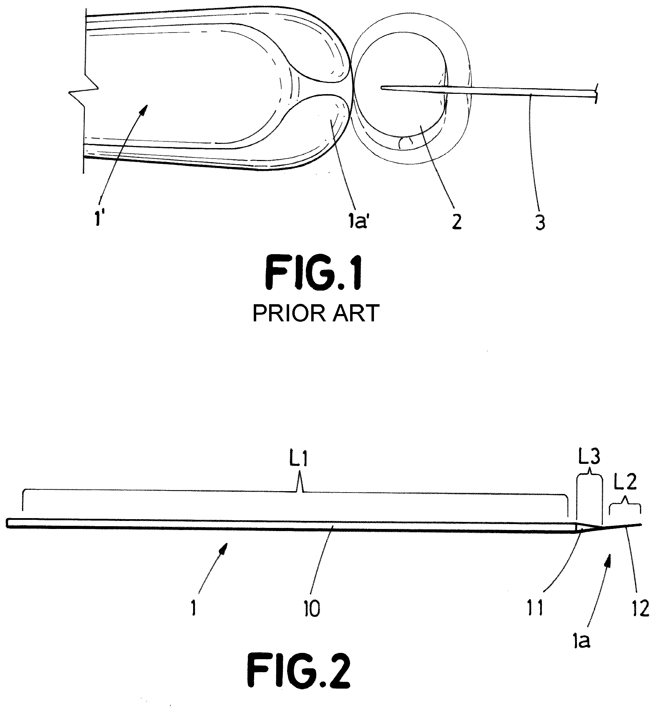

In that sense, as observed in Figure 1, the conventional holding pipettes (1') have a rounded working end (1a') and only hold the oocyte (2) to enable perforating its membrane and inserting the spermatozoid with the injection pipette (3), after a previous step of cytoplasm aspiration performed with said injection pipette (3).

Unlike the prior art, the holding pipette (1) of the invention works without aspiration, for which, as shown in Figures 2 to 6, it is configured from a long and narrow cylindrical body (10) with a working end (1a) for holding the oocyte (2) by means of pressure, having a decreasing frustoconical portion (11) where the diameter thereof is significantly reduced until defining a small support tip (12) with a planar, straight, and angled configuration with respect to the cylindrical body (10), i.e., it is prolonged into a short segment forming an angle (a) between 20 and 30° with the axial axis of the cylindrical body (10).

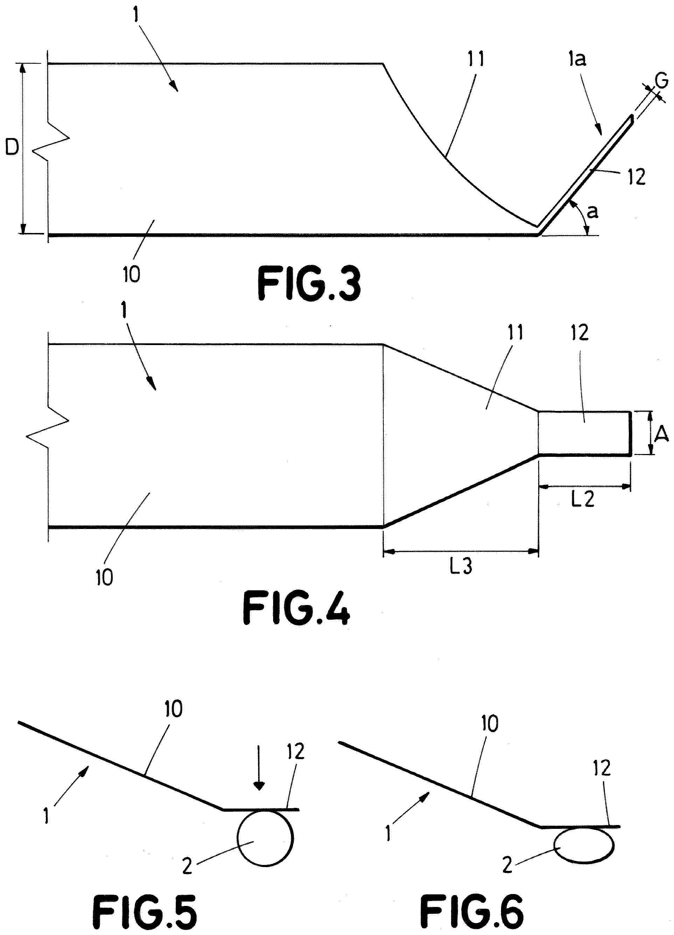

Figures 5 and 6 show the way in which said working end (1a) of the holding pipette (1) holds the oocyte (2) by slightly pressing on same, providing it with greater turgor to facilitate perforating the membrane with the injection pipette (3), which is not depicted in said drawings, but it will be the same as the one shown in Figure 1.

In the preferred embodiment, the cylindrical body (10) is about 5 cm in length (L1) and 500 microns in diameter (D), whereas the support tip (12) is between 400 and 1000 microns in length (L2).

Furthermore, the support tip (12) has also preferably a width (W) of 135 microns and a thickness (T) of 20 microns. Additionally, the decreasing frustoconical portion (11) has a length (L3) of 600 microns.

Having sufficiently described the nature of the present invention, as well as the manner of putting it into practice, it is not considered necessary to expand on this explanation so that one skilled in the art will understand its scope and the advantages derived from it, hereby stating that within its essential nature, the invention may be carried out to practice in other embodiments which differ in detail from that indicated by way of example and which will likewise attain the protection that is sought provided that its fundamental principle is not altered, changed, or modified.

## Claims

1. A pipette for holding oocytes in intracytoplasmic sperm injection procedures which, applicable to perform said holding without prior cytoplasm aspiration, is **characterized by** being configured from a long and narrow cylindrical body (10) provided with a working end (1a) for holding the oocyte (2) by means of pressure, having a decreasing frustoconical portion (11) where the diameter thereof is significantly reduced until defining a small support tip (12) having a planar, straight, and angled configuration with respect to the cylindrical body (10).

2. The pipette for holding oocytes in intracytoplasmic sperm injection procedures according to claim 1, **characterized in that** the support tip (12) is prolonged into a short segment forming an angle (a) between 20 and 30° with the axial axis of the cylindrical body (10).

3. The pipette for holding oocytes in intracytoplasmic sperm injection procedures according to claim 1 or 2, **characterized in that** the cylindrical body (10) is 5 cm in length (L1) and 500 microns in diameter (D), and the support tip (12) is between 400 and 1000 microns in length (L2).

4. The pipette for holding oocytes in intracytoplasmic sperm injection procedures according to claim 3, **characterized in that** the support tip (12) has a width (W) of 135 microns and a thickness (T) of 20 microns.

5. The pipette for holding oocytes in intracytoplasmic sperm injection procedures according to claim 3 or 4, **characterized in that** the decreasing frustoconical portion (11) has a length (L3) of 600 microns.
